# EUROPEAN PATENT SPECIFICATION

(11) **EP 1 495 109 B1**
(45) Date of publication and mention of the grant of the patent: **28.12.2005**
(21) Application number: 03746006.0
(22) Date of filing: 04.04.2003
(51) Int. Cl.: C12N 1/04, C12N 1/20, A61K 35/74, A23C 9/123

(54) **METHOD OF PREPARING A PROBIOTIC PREPARATION**
VERFAHREN ZUR HERSTELLUNG EINER PROBIOTISCHEN PRÄPARATION
PROCEDE DE PRODUCTION DE PREPARATION PROBIOTIQUE

(30) Priority: 04.04.2002 NL 1020301
(43) Date of publication of application: 12.01.2005
(73) Proprietor: Winclove Bio Industries B.V., 1032 KJ Amsterdam (NL)
(72) Inventor: PEKELHARING, Peter, Remco, NL-1012 CX Amsterdam (NL); VAN LEEUWEN, Abraham, Benjamin, Jacobus, NL-1113 LL Diemen (NL)
(74) Representative: Kupecz, Arpad
(86) International application number: PCT/NL2003/000255
(87) International publication number: WO 2003/085097

(56) References cited:
- EP-A- 0 861 905
- EP-A- 1 177 794
- EP-A- 1 243 181
- WO-A-97/29640
- WO-A-98/27824
- FR-A- 2 750 298
- GB-A- 2 193 875
- US-A- 5 902 578

## Description

The present invention relates to a method of manufacturing a probiotically active preparation.

Such preparations and methods of their manufacture are generally known, and often comprise culturing a probiotic microorganism on a medium containing a liquid substrate, such as a dairy product. The preparations are taken for the prevention of, or to promote the recovery from intestinal complaints. More specifically, WO 97/29640 to Hakalehto E. et al disclose a method of souring milk, by adding a microorganism to milk. It is disclosed to use a container having a surface in which a pH is incorporated, allowing a consumer to see that the product is ripened/is ready for consumption.

Due to the central and industrial-scale production, the probiotic microorganism in the preparation is not quite effective when consumed by the user.

It is the object of the present invention to provide a method by which a probiotic preparation can be manufactured with a more effectively working probiotic microorganism.

To this and the method according to the present invention is characterised in that a composition comprising a probiotic microorganism, a metabolisable substrate and a growth indicator is contacted with a non-sterile aqueous liquid and is incubated for at least five minutes and until the growth indicator shows a change in colour, the preparation for consumption containing the probiotic microorganism in a non-dormant state.

Incubation of the probiotic microorganism in the presence of substrate induces the microorganism, which will usually be dormant, to enter a dividing phase. The composition can subsequently be taken by a user and the microorganisms will be better able to carry out their favourable activity in the gastrointestinal tract. Duration of incubation is suitably at least 5 minutes, preferably at least 20 minutes and more preferably at least 1.5 hours. In other words, incubation takes place as long as is necessary for activation after dormancy and preferably for one or several divisions of the probiotic microorganism to take place. The method according to the present invention also makes it possible to use water of a biologically dubious quality. Aided by the lowering of the pH caused by fermentation, the probiotic composition inhibits, inactivates, or even kills possible pathogenic microorganisms present in the water. In practice, incubation takes place under non-sterile conditions, such as in a non-sterile container, and in a non-sterile aqueous liquid. After incubation the preparation is ready for consumption and, in contrast with known commercial preparations that are packaged after manufacture, this preparation is in practice not packaged. "Substrate" is understood to be a substrate that can be metabolised by the probiotic microorganism and comprises, for example, maltodextrin, fructo-oligosaccharides or the like. Optionally traces of minerals such as potassium, manganese and magnesium may also be present to promote the growth of the probiotic microorganism. When referring in the present application to an aqueous liquid, this is understood to be any liquid containing sufficient water to enable the probiotic microorganism to divide. If the composition comprises several components, among which in particular one or several probiotic microorganisms and substrate, these may be packaged separately in the form of a kit. A first package may, for example, comprise one or several probiotic microorganisms together with an inert carrier, and the second packet may comprise the substrate. Both packets are added to the aqueous liquid. However, preferably the composition comprises a combination, such as a mixture, of substrate and probiotic microorganism, which combination therefore requires only one single packet. Preferably, the composition is packaged as "unit doses", where the packet such as a sachet, contains an amount of composition to be contacted with a previously determined amount of aqueous liquid. In this way a dose of the preparation ready for consumption is produced. As mentioned above, the substrate and the microorganism for the composition may be packaged separately, that is to say each may be packaged as a unit dose. The probiotic preparation according to the invention is usually a drink. When using the term "non-sterile", in the present invention, the probiotic micro-organisms of this preparation are left out of consideration.

The non-sterile aqueous liquid is preferably water, in particular water that of itself does not contain any organic carbon source as substrate, such as mineral water, tap water or natural water.

Thanks to the method according to the invention, the non-sterile aqueous liquid can be made better suitable and can even be made safe for consumption. This can also be done indirectly, if the water is used to wash fresh vegetables and fruit, which in some countries holds an increased risk of causing intestinal disorders, such as diarrhoea. The method according to the invention has been shown to reduce the bacterial count of potentially harmful microorganisms such as *E. coli*. For this particular application the incubation period is preferably longer than 6, more particularly longer than 10 hours. For this application it is not important whether the probiotic microorganisms have after incubation returned to a stationary, dormant condition, though in accordance with the invention, it is still preferred for them not to be in a dormant condition.

According to an important embodiment, the composition comprises a growth indicator and incubation lasts until the growth indicator shows a change in colour.

In this way a variety of factors can be taken into account such as the occasionally changing incubation conditions, viability of the probiotic microorganism, etc. A growth indicator shows at least that the microorganism has come out of dormancy and when consumed is thus able to immediately produce a favourable probiotic effect. In the case of reducing the bacterial count of possible pathogenic microorganisms it is preferred to use a growth indicator that exhibits a delayed change.

The growth indicator used in a preferred embodiment is a pH indicator.

In this way a suitable length of incubation is shown. This is of particular importance in conditions where the incubation temperature cannot be adjusted or regulated. The indicator shows when the microorganisms have divided sufficiently often or the pH of the water is sufficiently lowered to kill possibly present pathogenic microorganisms. According to an alternative embodiment, the growth indicator used may be a substrate that through cleavage produces a dye, as is well known in the field of microbiology. An example of such a growth indicator is an indicator substrate for beta-galactosidase, such as orthonitrophenyl-b-D-galactopyranoside. Although this works well and will be preferred in buffered solutions, from an economical viewpoint and for many practical applications it will be preferred to use a pH indicator in non-buffered or only slightly buffered solutions such as water. The term buffered in this case refers to buffered in the pH range that in the absence of the buffering substance can be attained by the probiotic microorganism prior to the formation of acid and after the formation of acid.

Preferably a pH indicator is used having a point of change at pH = 5 or lower, more preferably at a pH between 3.5 and 4.5.

At such a pH, effective killing or inactivation of possibly present pathogenic microorganisms is achieved. A change to such a pH also provides a good indication of sufficient division of the probiotic microorganism. When choosing an indicator around pH 4 or lower, the change will take place later, which is favourable for an application such as the reduction of the bacterial count of possibly pathogenic microorganisms.

It is preferred to use as the probiotic microorganism at least one probiotic microorganism chosen from *Lactobacillus, Lactococcus, Propionibacterium, Pediococcus, Bifidobacterium, Enterococcus,* and *Streptococcus.*

Microorganisms such as, for example, *Lactobacillus acidophilus, Lactobacillus casei, Lactobacillus salivarius, Lactoba cillus plantarum, Lactococcus lactis, Propionibacterium freudenreichii, Pediococcus acidilactici, Bifidobacterium lactis, Enterococcus faecium,* and *Streptococcus lactis,* make it possible to produce an effective probiotic preparation. It is, of course, possible to use a mixture of more than one probiotic microorganism, taking care that each probiotic microorganism has a substrate at its disposal to enable it to bring the microorganism into a dividing (log-) phase.

According to a preferred embodiment the composition is a dry composition.

In contrast with many of the usual commercially available probiotic preparations, such a composition can be stored for a long time, and takes up little space. In addition the shelf life is considerably extended without jeopardising the effectiveness. This is especially the case in the presence of a growth indicator. Within the scope of the present invention, "dry" means in the absence of so much water as to render the microorganism dormant. The manufacture of dry compositions comprising microorganisms is well-known in the art. It may be realised, for example, by means of spray-drying or lyophilisation. Such a composition requires no cool storage. The metabolisable substrate may be mixed with the microorganism either prior to but preferably subsequent to drying the microorganism. The composition may comprise a carrier such as cornstarch.

The invention also relates to a probiotically active composition, which is characterised in that the composition comprises a probiotic microorganism, a metabolisable substrate for the microorganism and a growth indicator, and optionally a pharmaceutically acceptable carrier or excipient.

Preferably the composition is a dry composition.

In contrast with known probiotic compositions currently on the market, such a composition has a very long shelf life.

It will be obvious to the ordinary person skilled in the art that the invention may be applied in a variety of ways. For example, it is possible to carry a preparation made up with water in a container such as flat flask against the body to provide an environment of elevated temperature enabling the probiotic microorganism to grow more quickly and allowing the ready preparation to be used sooner.

The invention will now be elucidated with reference to the following examples.

### Example 1

As model pathogen use was made of *E. coli* ATCC 25922 cultured in Brain Heart Infusion (BHI)-broth by incubating for 18 hours at 37°C. This provided mature cultures comprised of approximately 10⁸ colony-forming units (cfu) per ml. From this solution a dilution series was derived in a sterile peptone physiological (pfz)) solution.

Subsequently various samples (100 ml) of Amsterdam tap water were inoculated with different *E. coli* ATCC 25922 concentrations, to wit:

| Sample | Initial conc. *E*. *coli* (cfu/ml) |
|---|---|
| A | 3.8*10² |
| B | 3.8*10³ |
| C | 3.8*10⁴ |
| D | 3.8*10⁵ |
| E | 3.8*10⁶ |
| F (control) | 4.0*10⁶ |
| G (control) | 4.0*10⁴ |

After that a known concentration (4.8 * 10⁷ cfu/ml) of the probiotic composition was added, consisting of 1% Lactobacilli (*L. plantarum, L. casei, L. acidophilus* and *L. salivarius* at approximately equal quantities), 20% maltodextrin, 5% dextrose, 5% mineral mix (equal quantities of potassium chloride, magnesium sulphate and manganese sulphate), 64% cornstarch (carrier) and 5% caranthopowder as a colour indicator.
In the course of the experiment all the water samples were left at 37°C (without shaking).

After 6 and 24 hours the bacterial count of *E. coli* ATCC 25922 was determined. Using sterile pfz decimal dilutions of the samples were made, which were subsequently incubated on media suitable for the respective microorganism. An *E. coli* test was carried out using Eosine Methylene Blue agar (EMB, Petri dishes). After 24 hours incubation (aerobic) at 37°C these dishes were evaluated (counted). The test for the Lactobacilli was performed using Man, Rogosa, Sharp Agar (MRSA, Petri dishes). These plates were evaluated after 48 incubation (anaerobic) at 37°C.

In addition the pH was measured at t=0, t=6 and t=24 hours.

### Control (samples F and G):

As control experiment, no probiotic bacteria were added to two different starting concentrations of *E. coli* (10⁶ and 10⁴, control F and G, respectively) whereas the other components of the composition such as the nutrients, were added. In order to determine the influence of acid production only, the pH was adjusted by acidifying (0.1 M HCl) to the same pH that was measured in the experiment with the probiotic preparation.

At different points in time a sample was taken to determine the bacterial count of *E. coli*. At t=6 hours the bacterial count of the probiotic bacteria of experiment A was determined.

### Results

The results of the study are shown in the tables and graph below.

**Table 1.**

| Total bacterial count of *E. coli* ATCC 25922 present in the various water samples at three points in time (expressed in cfu/ml). | | | |
|---|---|---|---|
| sample | Time (hours) | | |
| | 0 | 6 | 24 |
| A | 3.8*10² | 5.0*10² | 0 |
| B | 3.8*10³ | 1.5*10⁴ | 1 |
| C | 3.8*10⁴ | 6.2*10⁵ | 6.0*10² |
| D | 3.8*10⁵ | 4.8*10⁷ | 2.8*10³ |
| E | 3.8*10⁶ | 1.0*10⁸ | 6.0*10² |
| F (control) | 4.0*10⁶ | 6.0*10⁷ | 8.0*10⁷ |
| G (control) | 4.0*10⁴ | 2.2*10⁴ | 1.0*10⁷ |

For the probiotic microorganisms relating to experiment A and 6 hours, an increase to 1.1 x 10⁸ cfu/ml was measured. The pH values were 6.3, 4.1 and 3.7 on t=0, t=6 and t=24, respectively. After 6 hours a colour change was observed from purple-pink to deep pink.

### Conclusion

The probiotic preparation inhibits the growth of *E. coli* ATCC 25922 in water. Incubating an initial concentration of 3.8*10² cfu/ml of *E. coli* for 24 hours with the probiotic preparation, even resulted in no *E. coli* being detected in the water. The two control experiments in which the pH is adjusted but no probiotic bacteria are added show that *E. coli* ATCC 25922 is not inhibited or that there is even some growth. This shows that the inhibition of *E. coli* ATCC 25922 is not or not solely caused by the pH drop but also by another, probiotic microorganism-dependant factor.

### Example 2

In an experiment similar to that of Example 1 the influence was studied of a probiotic preparation on a number of pathogens present (through addition) in both tap water from Wageningen (town in the Netherlands) and demineralised water. The probiotic composition was the same as in Example 1.

### Material and methods

### Micro-organisms

The following three pathogens were used in the study:
1. *Shigella flexneri* (strain collection University of Wageningen, the Netherlands);
*2. Salmonella typhimurium* (strain collection University of Wageningen, the Netherlands);
*3. Escherichia coli* (strain H03-50 from the RIVM, Lelystad, the Netherlands, isolated from faeces of a patient suffering from (bloody) diarrhoea).

For use the strains were cultured in Brain Heart Infusion (BHI)-broth by incubation for 18 hours at 37°C. In this way mature cultures were obtained of approximately 10⁹ colony-forming units (cfu) per ml.

### Water

Two kinds of water were used in this study:
- tap water (tap water - suitable for consumption);
- demineralised water (demi-water).

Measuring flasks were filled with water (100 ml water per flask) and sterilised for 15 minutes at 121°C. Thus the water will be contaminated with the test pathogens only.

### Experimental set-up

To the various measures of water, with the exception of the blank series, 6.0 grams of the probiotic preparation (initial concentration 3 * 10⁷ cfu/ml) were added. These samples were subsequently inoculated with the pathogenic bacteria (1 type of bacteria per 100 ml water) in different initial concentrations (10⁴ cfu/ml and 10⁶ cfu/ml). The 10⁶ cfu/ml water was obtained by using 0.1 ml of a mature culture to aseptically inoculate 100 ml of water in a measuring flask. To obtain an initial concentration of 10⁴ cfu/ml of water, a mature culture was (decimally) diluted with sterile peptone physiological (pfz)-solution, after which 0.1 ml of a suitable dilution was used to inoculate 100 ml water in a measuring flasks.

At t=0 (after addition of probiotica and inoculation with a bacterium), t=6, t=11 and t=24 hours samples were taken to be examined for the number of pathogens. Using sterile pfz decimal dilutions of the samples were made which were subsequently examined (in duplicate) in a manner known in itself, with the aid of suitable media. *Shigella* and *Salmonella* were tested for with Violet Red Bile Glucose (VRBG) agar (cast plates), *E. coli* was tested for with Violet Red Bile Lactose (VRBL) agar (cast plates). The plates were assessed (counted) after 24 hours incubation at 37°C. As control, 0.1 ml of a suitable dilution of the samples was spread on the selective medium Xylose Lysine Dextrose (XLD) agar to determine the typical colony appearance of *Salmonella* and *Shigella* (in order to exclude contamination).

In the course of the experiment all the water samples were set aside at 37°C (without shaking).

### Results

The results of the study are presented in Table 2 below. Once again a colour change was observed, indicating that the probiotic microorganisms were active.

**Table 2.**

| Numbers of pathogenic bacteria present in the various water samples at four points in time (expressed as log cfu/ml). | | | | | | | | | |
|---|---|---|---|---|---|---|---|---|---|
| **Samples in demi- water** | **t=0** | **t=6** | **t=11** | **t=24** | **Samples In tap water** | **t=0** | **t=6** | **t=11** | **t=24** |
| *Shigella* | 3.8 | 3.5 | 3.2 | <0.5 | *Shigella* | 3.7 | 1.5 | <0.5 | <0.5 |
| *Shigella* | 5.4 | 5.5 | 5.5 | <0.5 | *Shigella* | 6.0 | 3.0 | <0.5 | <0.5 |
| *Shigella* (blank) | 6.0 | 4.8 | 4.6 | 4.4 | *Shigella* (blank) | 5.9 | 3.3 | 2.9 | 0.7 |
| *Salmonella* | 3.9 | 1.6 | 1.0 | 0.9 | *Salmo- nella* | 4.0 | 3.2 | <0.5 | <0.5 |
| *Salmonella* | 6.0 | 4.6 | 2.2 | <0.5 | *Salmo- nella* | 6.2 | 5.2 | 2.9 | <0.5 |
| *Salmonella* (blank) | 5.9 | 6.0 | 6.1 | 6.0 | *Salmo- nella* (blank) | 6.0 | 3.9 | 4.0 | 3.9 |
| *E. coli* | 3.9 | 2.1 | 2.3 | <0.5 | *E. coli* | 3.7 | 3.0 | 0.9 | <0.5 |
| *E. coli* | 6.1 | 5.4 | 5.2 | 0.5 | *E. coli* | 5.9 | 5.3 | 2.0 | <0.5 |
| *E. coli* (blank) | 6.0 | 5.7 | 5.8 | 5.5 | *E. coli* (blank) | 6.1 | 4.6 | 4.4 | 3.7 |

### Conclusion

The addition of the probiotic preparation to both tap water and demi-water, in which pathogenic bacteria are present, was shown to have an inactivating effect on the pathogens. After 24 hours the pathogens are no longer detectable in tap water. At that moment low numbers of the bacteria *Salmonella* and *E. coli* are still detectable in demi-water.

## Claims

1. A method of manufacturing a probiotically active preparation, **characterised in that** a composition comprising a probiotic microorganism, a metabolisable substrate and a growth indicator is contacted with a non-sterile aqueous liquid and is incubated for at least five minutes and until the growth indicator shows a change in colour.

2. A method according to claim 1, **characterised in that** the non-sterile aqueous liquid is water.

3. A method according to claim 1 or 2, **characterised in that** the growth indicator used is a pH indicator.

4. A method according to claim 3, **characterised in that** a pH indicator is used having a turning point at pH = 5 or lower, preferably at a pH between 3.5 and 4.5.

5. A method according to one of the preceding claims, **characterised in that** as the probiotic microorganism at least one probiotic microorganism chosen from *Lactobacillus, Lactococcus, Propionibacterium, Pediococcus, Bifidobacterium, Enterococcus,* and *Streptococcus* is used.

6. A method according to one of the preceding claims, **characterised in that** the composition is a dry composition.

7. A probiotically active composition, **characterised in that** the composition comprises a probiotic microorganism, a metabolisable substrate for the microorganism and a growth indicator, and optionally a pharmaceutically acceptable carrier or excipient.

8. A composition according to claim 7, **characterised in that** it is a dry composition.

## Revendications

1. Procédé de fabrication d'une préparation probiotiquement active, **caractérisé en ce qu'**une composition comprenant un micro-organisme probiotique, un substrat métabolisable et un indicateur de croissance sont mis en contact avec un liquide aqueux non stérile et sont incubés pendant au moins cinq minutes et jusqu'à ce que l'indicateur de croissance montre un changement de couleur.

2. Procédé selon la revendication 1, **caractérisé en ce que** le liquide aqueux non stérile est l'eau.

3. Procédé selon la revendication 1 ou 2, **caractérisé en ce que** l'indicateur de croissance utilisé est un indicateur de pH.

4. Procédé selon la revendication 3, **caractérisé en ce qu'**un indicateur de pH est utilisé ayant un point de virage à pH = 5 ou moins, de préférence à un pH compris entre 3,5 et 4,5.

5. Procédé selon l'une des revendications précédentes, **caractérisé en ce que** comme micro-organisme probiotique, au moins un micro-organisme probiotique choisi parmi *Lactobacillus, Lactococcus,* *Propionibacterium, Pediococcus, Bifidobacterium, Enterococcus,* et *Streptococcus* est utilisé.

6. Procédé selon l'une des revendications précédentes, **caractérisé en ce que** la composition est une composition sèche.

7. Composition probiotiquement active, **caractérisé en ce que** la composition comprend un micro-organisme probiotique, un substrat métabolisable pour le micro-organisme et un indicateur de croissance, et facultativement un véhicule ou excipient pharmaceutiquement acceptable.

8. Composition selon la revendication 7, **caractérisé en ce que** c'est une composition sèche.

## Patentansprüche

1. Verfahren für die Herstellung einer probiotisch aktiven Zubereitung, **dadurch gekennzeichnet, dass** eine Zusammensetzung umfassend einen probiotischen Mikroorganismus, ein metabolisierbares Substrat und einen Wachstumsindikator mit einer nichtsterilen wässrigen Flüssigkeit in Kontakt gebracht und mindestens fünf Minuten lang und bis der Wachstumsindikator eine Farbänderung anzeigt, inkubiert wird.

2. Verfahren nach Anspruch 1, **dadurch gekennzeichnet, dass** die nichtsterile wässrige Flüssigkeit Wasser ist.

3. Verfahren nach Anspruch 1 oder 2, **dadurch gekennzeichnet, dass** der verwendete Wachstumsindikator ein pH-Indikator ist.

4. Verfahren nach Anspruch 3, **dadurch gekennzeichnet, dass** ein pH-Indikator verwendet wird, der einen Umschlagpunkt bei pH = 5 oder weniger, bevorzugt bei einem pH-Wert zwischen 3,5 und 4,5 aufweist.

5. Verfahren nach einem der vorhergehenden Ansprüche, **dadurch gekennzeichnet, dass** als probiotischer Mikroorganismus mindestens ein probiotischer Mikroorganismus ausgewählt unter *Lactobacillus, Lactococcus, Propionibacterium, Pediococcus, Bifidobacterium, Enterococcus* und *Streptococcus* verwendet wird.

6. Verfahren nach einem der vorhergehenden Ansprüche, **dadurch gekennzeichnet, dass** die Zusammensetzung eine trockene Zusammensetzung ist.

7. Probiotisch aktive Zusammensetzung, **dadurch gekennzeichnet, dass** die Zusammensetzung einen probiotischen Mikroorganismus, ein metabolisierbares Substrat für den Mikroorganismus und einen Wachstumsindikator und wahlweise einen pharmazeutisch akzeptablen Träger oder ein pharmazeutisch akzeptables Vehikel umfasst.

8. Zusammensetzung nach Anspruch 7, **dadurch gekennzeichnet, dass** sie eine trockene Zusammensetzung ist.
